# FASCICULE DE BREVET EUROPEEN

(11) **EP 2 869 781 B1**
(45) Date de publication et mention de la délivrance du brevet: **23.03.2022**
(21) Numéro de dépôt: 13744685.2
(22) Date de dépôt: 04.07.2013
(51) Int. Cl.: A61C 1/08, A61B 5/00, A61B 1/06, A61B 1/24

(54) **DISPOSITIF D'ECLAIRAGE PEROPERATOIRE**
PEROPERATIVE BELEUCHTUNGSEINRICHTUNG
PEROPERATIVE ILLUMINATION DEVICE

(30) Priorité: 06.07.2012 FR 1256555
(43) Date de publication de la demande: 13.05.2015
(73) Titulaire: SOCIETE POUR LA CONCEPTION DES APPLICATIONS DES TECHNIQUES ELECTRONIQUES, 33700 Merignac (FR)
(72) Inventeur: MAZUIR, Alain, F-83470 Saint Maximin La Sainte Beaume (FR); DIERAS, Francis, F-33000 Bordeaux (FR); REGERE, Pascal, F-33290 Blanquefort (FR)
(74) Mandataire: Cabinet Beau de Loménie
(86) Numéro de dépôt international: PCT/FR2013/051589
(87) Numéro de publication internationale: WO 2014/006337

(56) Documents cités:
- EP-A1- 1 269 909
- DE-U1-202004 001 189
- US-A1- 2006 152 926
- US-A1- 2010 053 933
- US-B1- 6 386 866

## Description

Le présent exposé concerne un dispositif d'éclairage dentaire comprenant au moins deux sources lumineuses d'appoint solidaire d'un instrument d'appoint.

### ETAT DE LA TECHNIQUE ANTERIEURE

Conventionnellement, une zone dentaire prédéterminée à travailler chez un patient est éclairée par un scialytique, qui fournit un éclairage d'ambiance pouvant s'avérer insuffisant pour bien des actes de dentisterie.

Tel peut notamment être le cas pour un acte de détartrage, lorsque ce dernier requiert l'usage de révélateurs colorés destinés à pénétrer le tartre pour permettre un repérage de tartre par fluorescence sous un éclairage particulier. Notamment, il est d'usage de recourir à des révélateurs qui émettent par fluorescence dans le jaune lorsque la substance révélatrice est exposée à un éclairage dans le bleu.

Dans ce cas, un éclairage de la zone dentaire à travailler par seulement la lumière d'ambiance fournie par le scialytique ne permet pas d'obtenir l'éclairage bleu requis pour provoquer un niveau de fluorescence du révélateur suffisant pour permettre au dentiste de repérer le tartre avec précision.

Cette absence de précision conduit bien souvent le dentiste à réaliser un sur-traitement, en attaquant de façon plus importante que nécessaire l'émail de la dent.

Par ailleurs, le dentiste a besoin de visualiser la zone dentaire avec un éclairage sensiblement dans le blanc pour pouvoir deviner avec précision les structures anatomiques et les surfaces à travailler des dents car, autrement, le risque de réaliser un sur-traitement est là encore grand.

Il existe par conséquent un besoin pressant pour la mise au point d'un dispositif d'éclairage dentaire permettant de réduire les risques de sur-traitement lors d'un acte de dentisterie réalisé à l'aide d'une substance révélatrice fluorescente.

US2007/121786 divulgue une pièce à main dentaire comprenant plusieurs sources lumineuses d'excitation, sélectionnables , émettant à des longueurs d'ondes différentes, et une lumière d'illumination. La lumière d'excitation et la lumière d'illumination peuvent être émises simultanément ou séparément. Leur combinaison permet ainsi la détection de tartre sur les dents. Parmi les lumières d'excitation possible figure la lumière bleu proche des ultra-violets et ayant une longueur d'onde entre 355 et 455 nm. La lumière d'illumination est une lumière blanche de longueur d'onde comprise entre 400 et 700 nm.

### PRESENTATION DE L'INVENTION

L'invention défini un dispositif d'éclairage dentaire selon la revendication et un procédé de configuration de ce dispositif d'éclairage selon la revendication 11. Un premier aspect du présent exposé concerne un dispositif d'éclairage dentaire, comprenant au moins un système d'éclairage configuré pour éclairer une zone dentaire prédétermine avec, sur une première plage spectrale qui est inférieure à une longueur d'onde de référence comprise entre 405 nm et 475 nm, une première valeur moyenne d'éclairement E1 et, sur une deuxième plage spectrale supérieure à cette longueur d'onde de référence, une deuxième valeur moyenne d'éclairement E2, lequel système d'éclairage est configuré pour fonctionner selon au moins un premier mode d'éclairage, dans lequel le rapport de la première valeur moyenne d'éclairement E1 sur la deuxième valeur moyenne d'éclairement E2 est inférieur à 12.

On entend dans ce document par « valeur moyenne d'éclairement » la définition radiométrique conventionnelle de ce terme, à savoir dans le cas présent la puissance lumineuse moyenne reçue par unité de surface, cette valeur étant calculée par intégration sur l'ensemble de la plage spectrale considérée (i.e. la première ou la deuxième plage spectrale dans le cadre de l'invention).

Sauf indication contraire, on utilisera dans la suite de ce document indifféremment les termes « valeur d'éclairement » et « valeur moyenne d'éclairement ».

Un dispositif d'éclairage selon ce premier aspect comprend ainsi un système d'éclairage qui est configuré pour éclairer une zone dentaire prédéterminée dans une première couleur formée par émission d'une ou plusieurs plus petite(s) longueur(s) d'onde (pour éclairer ladite zone avec une première température de couleur plus élevée). En effet, ce système d'éclairage est configuré pour émettre une première pluralité de rayons lumineux dans une première plage spectrale qui est inférieure à une longueur d'onde de référence comprise entre 405 nm et 475 nm (nanomètres).

Ainsi, les rayons lumineux de cette première pluralité sont émis par le système d'éclairage avec des longueurs d'ondes d'émission respectives qui sont, chacune, inférieures à cette longueur d'onde de référence. Les rayons lumineux de cette première pluralité sont conformés par le système d'éclairage pour éclairer la zone dentaire prédéterminée dans la première couleur avec une première valeur d'éclairement E1.

Cette première valeur d'éclairement E1 correspond donc au premier flux, qui est transporté par l'ensemble des rayons lumineux de la première pluralité (i.e. par l'ensemble des rayons lumineux qui, d'une part, impactent la zone dentaire prédéterminée et qui, d'autre part, sont, chacun, émis avec une longueur d'onde d'émission propre qui est inférieure à la longueur d'onde de référence), divisé par la superficie S de cette zone dentaire.

Compte-tenu de la valeur de la longueur d'onde de référence, qui peut arbitrairement être choisie en tout point de l'intervalle spectral [405 nm ; 475 nm], ladite première couleur peut être, par exemple, du bleu, de l'indigo, du violet, de l'ultraviolet, ou autre. Dès lors, dans le cadre du présent exposé, cette première couleur sera génériquement dénommée « couleur bleue » ou « dans le bleu » par abus de langage.

Par ailleurs, le système d'éclairage d'un dispositif d'éclairage selon le premier aspect précité est configuré pour en outre éclairer la zone prédéterminée avec une deuxième couleur formée par émission d'une ou plusieurs plus grande(s) longueur(s) d'onde (pour en outre éclairer ladite zone avec une deuxième température de couleur plus faible). En effet, ce système d'éclairage est configuré pour émettre une deuxième pluralité de rayons lumineux dans une deuxième plage spectrale qui est supérieure à la longueur d'onde de référence, qui, rappelons-le, est comprise entre 405 nm et 475 nm.

Ainsi, les rayons lumineux de cette deuxième pluralité sont émis par le système d'éclairage avec des longueurs d'ondes d'émission respectives qui sont, chacune, supérieures à cette longueur d'onde de référence. Les rayons lumineux de cette deuxième pluralité sont conformés par le système d'éclairage pour éclairer la zone dentaire prédéterminée dans la deuxième couleur avec une deuxième valeur d'éclairement E2.

Cette deuxième valeur d'éclairement E2 correspond donc au deuxième flux, qui est transporté par l'ensemble des rayons lumineux de la deuxième pluralité (i.e. par l'ensemble des rayons qui, d'une part, impactent la zone dentaire prédéterminée et qui, d'autre part, sont, chacun, émis avec une longueur d'onde d'émission propre qui est supérieure à la longueur d'onde de référence), divisé par la superficie S de cette zone dentaire.

Compte-tenu de la valeur de la longueur d'onde de référence, qui peut arbitrairement être choisie en tout point de l'intervalle spectral [405 nm ; 475 nm], ladite deuxième couleur peut être, par exemple, du blanc chaud léger, du blanc plus chaud, ou une autre couleur davantage monochromatique. Dès lors, dans le cadre du présent exposé, cette deuxième couleur sera génériquement dénommée « couleur plus chaude que le bleu » par abus de langage.

Ainsi, un dispositif d'éclairage dentaire selon le premier aspect précité est capable d'éclairer « dans le bleu » la zone dentaire prédéterminée, avec une première valeur d'éclairement E1, ce qui permet de rendre plus aisée la fluorescence d'un révélateur qui doit absorber des rayonnements dans ce bleu pour réémettre par fluorescence des rayons lumineux, en particulier dans le jaune.

Dès lors, cet éclairage « dans le bleu » contribue à mieux révéler le tartre à l'aide d'un révélateur adapté. Néanmoins, cet éclairage dans le bleu n'est pas suffisant à lui tout seul pour convenablement réduire les risques de sur-traitement lors d'un acte de dentisterie réalisé à l'aide d'une substance fluorescente. En effet, le praticien a besoin de pouvoir non seulement détecter les zones de tartre dans les dents mais également d'apprécier avec précision les structures anatomiques des dents pour réduire les risques de sur-traitement lors de l'acte de dentisterie. Or, comme indiqué plus haut, cette appréciation précise de ces structures anatomiques nécessite une observation avec une lumière sensiblement blanche de la zone dentaire.

Pour ce faire, un dispositif d'éclairage dentaire selon le premier aspect précité est en outre capable d'éclairer dans une « couleur plus chaude que le bleu » la zone dentaire prédéterminée, avec une deuxième valeur d'éclairement E2, ce qui permet d'obtenir une couleur d'ensemble, résultant de l'éclairage combiné de cette zone avec cumulativement la première couleur précitée « dans le bleu » et la deuxième couleur précitée « plus chaude que le bleu », qui est différente de cette première couleur et différente de cette deuxième couleur.

Il en résulte que le praticien peut observer les structures anatomiques des dents sous cette couleur d'ensemble, qui est différente de la première couleur « dans le bleu ». Néanmoins, l'obtention d'une telle couleur d'ensemble n'est pas suffisante en soi pour convenablement réduire les risques de sur-traitement lors de l'acte de dentisterie. En effet, encore faut-il que cette couleur d'ensemble ne soit pas trop saturée en première couleur « dans le bleu » pour permettre au dentiste d'apprécier avec précision les structures anatomiques des dents.

Dans cette perspective, un dispositif d'éclairage dentaire selon le premier aspect précité est spécialement configuré pour que cette couleur d'ensemble soit obtenue avec des proportions particulières de première et deuxième couleurs, qui permettent à cette couleur d'ensemble de ne pas trop être saturée en première couleur « dans le bleu ».

Il s'avère que l'œil humain est moins sensible dans le bleu que dans les autres couleurs, plus chaudes (de plus grande longueur d'onde que ce bleu), de sorte qu'il est possible de prévoir une première valeur d'éclairement E1 plus grande que la deuxième valeur d'éclairement E2 (et donc une valeur supérieure à 1 pour ledit rapport E1/E2), sans que ce surplus de couleur « dans le bleu » n'ait un impact significatif sur la couleur d'ensemble telle que perçue par le praticien. A condition toutefois qu'un tel surplus de couleur « dans le bleu » ne soit pas trop important.

Les travaux de recherche et développement menés par les inventeurs à l'origine du présent exposé ont permis de mettre en évidence que des conditions préférentielles d'observation étaient obtenues lorsque le rapport E1/E2, correspondant à la division de la première valeur d'éclairement E1 « dans le bleu » par la deuxième valeur d'éclairement E2 dans une couleur « plus chaude que le bleu », n'excède pas un plafond situé autour de 12 (autour de douze). Dès lors, le système d'éclairage d'un dispositif d'éclairage dentaire selon le premier aspect précité est configuré pour fonctionner selon au moins un premier mode d'éclairage (i.e. au choix, selon seulement ce premier mode ou selon également au moins un deuxième mode distinct dudit premier mode), dans lequel le rapport de la première valeur d'éclairement E1 sur la deuxième valeur d'éclairement E2 est inférieur à 12.

Ainsi, en définitive, les inventeurs à l'origine du présent exposé ont mis au point un dispositif d'éclairage dentaire selon le premier aspect précité, qui permet de réduire les risques de sur-traitement lors d'un acte de dentisterie réalisé à l'aide d'une substance révélatrice fluorescente.

En effet, selon ce premier aspect, le dispositif est apte à éclairer une zone dentaire prédéterminée « dans le bleu », afin de permettre au praticien de détecter par fluorescence les lieux dans la bouche du patient qui sont à traiter, et, cumulativement, dans une couleur « plus chaude que le bleu », afin de rééquilibrer la couleur d'ensemble qui est perçue par le praticien vers une couleur moins saturée en couleur bleu pour lui permettre d'apprécier avec davantage de précision les structures anatomiques des dents relativement auxdits lieux précités, un tel rééquilibrage étant adéquatement obtenu lorsque la proportion d'éclairage « dans le bleu » par rapport à la proportion d'éclairage dans une couleur « plus chaude que le bleu » n'excède pas une valeur plafond d'environ 12.

Par ailleurs, cette valeur plafond peut être optimisée dans certains cas, notamment pour tenir compte des niveaux de contrastes, entre portions d'émail entartrées et portion d'émail dépourvues de tartre, qui sont perçus par certains praticiens pour certains types de dents.

Ainsi, dans certains modes de réalisation, le rapport, dans le premier mode d'éclairage, de la première valeur d'éclairement E1 sur la deuxième valeur d'éclairement E2 peut être prévu inférieur à, au choix, au moins l'une quelconque des valeurs ci-après : 14 ; 12 ; 10 ; 8 ; 6 ; 4 ; 2.

Par ailleurs, cette valeur plafond pourra être optimisée à l'avenir, notamment pour tenir compte d'éventuelles d'autres contraintes soulevées par les clients et/ou des résultats d'éventuels travaux supplémentaires de développement. Des essais cliniques complémentaires réalisés par la déposante ont ainsi permis de vérifier que des performances optimales sont effectivement obtenues lorsque le rapport E1/E2 est inférieur à 12. Le respect de ce plafond haut particulier (i.e. de 12) offre des conditions préférentielles d'observation et permet en particulier au praticien d'apprécier avec précision les reliefs et les structures anatomiques des dents à traiter.

Dans certains modes de réalisation, le rapport, dans le premier mode d'éclairage, de la première valeur d'éclairement E1 sur la deuxième valeur d'éclairement E2 peut être prévu supérieur à, au choix, au moins l'une quelconque des valeurs ci-après : 1 ; 2 ; 4 ; 6 ; 8. Le fait de prévoir également un plafond bas pour le rapport E1/E2 peut permettre une optimisation de la première valeur d'éclairement E1 et favoriser notamment ainsi une fluorescence du lieux à travailler à l'aide d'un révélateur. Ces exemples de valeurs de plafond bas tiennent compte de la remarque susmentionnée, à savoir que l'œil humain est moins sensible dans le bleu que dans les autres couleurs primaires. Ils tiennent également compte du fait qu'une portion d'émail entartrée présente une réflectivité moindre que celle d'une portion d'émail dépourvue de tartre, moins rugueuse.

Dans certains modes de réalisation, le système d'éclairage peut être configuré pour, dans le premier mode d'éclairage, éclairer la zone dentaire prédéterminée avec la première valeur d'éclairement E1 qui est prévue supérieure à 1 W/m². Une telle valeur minimale pour la première valeur d'éclairement E1 peut notamment correspondre à une valeur seuil en-dessous de laquelle il devient moins aisé de percevoir la fluorescence des lieux à travailler.

Dans certains modes de réalisation, le système d'éclairage peut être configuré pour, dans le premier mode d'éclairage, éclairer la zone dentaire prédéterminée avec la première valeur d'éclairement E1 qui est prévue supérieure à, au choix, au moins l'une quelconque des valeurs ci-après : 5 W/m² ; 10 W/m²; 15 W/m² ; 20 W/m² ; 30 W/m² ; 40 W/m² ; 50 W/m². Au-dessus de cette valeur de 50 W/m², le rendement de l'émission par fluorescence de la substance révélatrice peut chuter drastiquement, tandis que le flux réfléchi par l'émail, notamment de manière spéculaire, peut atteindre un niveau de puissance causant un éblouissement du praticien.

Dans certains modes de réalisation, le système d'éclairage peut être configuré pour, dans le premier mode d'éclairage, éclairer la zone dentaire prédéterminée avec la première valeur d'éclairement E1 qui est prévue inférieure à 300 W/m², voire inférieure à 100 W/m², par exemple, ce qui peut notamment permettre de tenir compte des problèmes relevés ci-avant, tout en rendant plus aisée une réduction des dimensions du système d'éclairage.

Dans certains modes de réalisation, le dispositif d'éclairage dentaire peut comprendre un système de commande couplé au système d'éclairage et configuré pour commander le fonctionnement dudit système d'éclairage selon au moins le premier mode d'éclairage. Ce système de commande peut faire partie intégrante du système d'éclairage, ou bien être dissocié de ce dernier.

Dans certains modes de réalisation, le dispositif d'éclairage dentaire peut être tel que la longueur d'onde de référence est comprise entre une valeur minimale MIN, qui supérieure à 405 nm, et une valeur maximale MAX, qui est inférieure à 475 nm. En effet, dans certaines applications, l'intervalle de tolérance défini par ces valeurs MIN et MAX peut être prévu plus petit que l'intervalle [405 nm ; 475 nm]. Cet intervalle de tolérance [MIN ; MAX] peut être prévu centré sur une longueur d'onde de fluorescence privilégiée d'un révélateur particulier qu'il serait envisagé d'utiliser.

Ainsi, par exemple, la valeur minimale MIN peut être choisie pour, au choix, être égale à l'une quelconque des valeurs ci-après : 410 nm ; 420 nm ; 430 nm ; 440 nm ; 450 nm ; 460 nm ; 470 nm. D'une manière analogue, la valeur maximale MAX peut être choisie pour, au choix, être égale à l'une quelconque des valeurs ci-après : 470 nm ; 460 nm ; 450 nm ; 440 nm ; 430 nm ; 420 nm ; 410 nm.

Il est dûment précisé que l'on peut choisir n'importe laquelle de ces exemples de valeurs MIN en combinaison de n'importe laquelle de ces exemples de valeurs MAX pour définir l'intervalle de tolérance précité, pourvu seulement que la valeur MIN choisie soit inférieure ou égale à la valeur MAX choisie. Par exemple, à titre illustratif, non limitatif, le dispositif d'éclairage dentaire peut être tel que la longueur d'onde de référence est comprise entre 440 nm et 460 nm.

Dans certains modes de réalisation, le dispositif d'éclairage dentaire peut être tel que la largeur de l'intervalle de tolérance [MIN ; MAX] précité (laquelle largeur correspond à la différence entre la valeur MAX et la valeur MIN) est inférieure à 20 nm. A titre d'exemple non limitatif, une telle valeur de largeur peut correspondre à la largeur d'un pic particulier d'émission d'une diode électroluminescente considérée, lorsqu'il est choisi d'équiper le système d'éclairage avec au moins une telle diode.

Dans certains modes de réalisation, le dispositif d'éclairage dentaire peut être tel que la première plage spectrale est comprise entre 370 nm et la longueur d'onde de référence. Ainsi, la première plage spectrale peut être entièrement contenue dans le spectre visible.

Dans certains modes de réalisation, le dispositif d'éclairage dentaire peut être tel que la deuxième plage spectrale est comprise entre la longueur d'onde de référence et 780 nm. Ainsi, la deuxième plage spectrale peut être entièrement contenue dans le spectre visible.

Dans certains modes de réalisation, le dispositif d'éclairage dentaire peut comprendre un scialytique, et le système d'éclairement peut comprendre au moins une source lumineuse d'ambiance solidaire de ce scialytique. Ainsi, la première valeur d'éclairement E1 et la deuxième valeur d'éclairement E2 peuvent, chacune, être au moins en partie obtenue à l'aide d'une telle source lumineuse d'ambiance.

Dans certains modes de réalisation, le dispositif d'éclairage dentaire peut comprendre un instrument d'appoint, et le système d'éclairage peut comprendre au moins une source lumineuse d'appoint solidaire de cet instrument d'appoint. Ainsi, la première valeur d'éclairement E1 et la deuxième valeur d'éclairement E2 peuvent, chacune, être au moins en partie obtenue à l'aide de cette au moins une source lumineuse d'appoint.

Dans certains modes de réalisation, le dispositif d'éclairage dentaire peut être tel que la première valeur d'éclairement E1 et la deuxième valeur d'éclairement E2 sont, chacune, obtenues intégralement à l'aide d'uniquement cette au moins une source lumineuse d'appoint. En d'autres termes, il n'est pas nécessaire de recourir à un éclairage supplémentaire, en particulier la source lumineuse d'ambiance d'un scialytique, pour obtenir les valeurs E1 et E2 désirées.

Dans certains modes de réalisation, le dispositif d'éclairage dentaire peut comprendre, cumulativement, le scialytique précité et l'instrument précité. Ainsi, la première valeur d'éclairement E1 et la deuxième valeur d'éclairement E2 peuvent, chacune, être obtenue à l'aide de l'éclairage cumulé fourni respectivement par ladite au moins une source lumineuse d'appoint solidaire de cet instrument et par la source lumineuse d'ambiance solidaire de ce scialytique.

Dans certains modes de réalisation, l'instrument d'appoint peut comprendre au moins une portion présentant des dimensions adaptées pour permettre l'introduction de cette portion dans la bouche d'un patient, ce qui peut permettre à ladite au moins une source lumineuse d'appoint de se trouver à proximité de la zone dentaire prédéterminée lorsqu'elle éclaire cette dernière.

Dans certains modes de réalisation, l'instrument d'appoint peut comporter un corps présentant une partie de fixation apte à coopérer avec un outil pour fixer ledit outil et le corps l'un par rapport à l'autre, et au moins un logement disposé au voisinage de la partie de fixation et dans lequel est montée ladite au moins une source lumineuse d'appoint. Un tel corps peut, par exemple, rendre plus aisée la préhension de l'instrument. Dès lors, l'instrument d'appoint peut servir d'outil de travail du praticien pour procéder à un acte de dentisterie particulier, tout en assurant l'éclairage souhaité pour permettre à ce praticien de mieux apprécier les structures anatomiques et les lieux à travailler. Ainsi, le regroupement de ces deux fonctions au sein d'un seul et même instrument permet de diminuer le nombre d'objets à manipuler en même temps lors de l'acte de dentisterie et, partant, de rendre plus aisé cet acte.

Dans certains modes de réalisation, ledit outil peut être un outil de détartrage (un détartreur), ce qui peut rendre plus précis et/ou aisé un acte de détartrage, l'outil pouvant être disposé à proximité de la zone dentaire éclairée par l'au moins une source lumineuse d'appoint.

Dans certains modes de réalisation, ledit instrument peut comprendre des moyens de préhension, par exemple un manche qui est solidaire du corps précité, lorsque l'instrument comprend un tel corps, et qui est apte à être saisi à pleine main, de manière à rendre plus aisé le déplacement dudit instrument.

Dans certains modes de réalisation, le système d'éclairage peut comprendre une pluralité de sources lumineuses d'appoint discrètes. En d'autres termes, un tel système d'éclairage peut comprendre, au choix, deux ou plus sources lumineuses d'appoint qui sont, chacune, distinctes les unes des autres, par exemple espacées les unes des autres. Une telle configuration peut rendre plus aisée l'obtention des conditions d'éclairage souhaitées.

Le système d'éclairage comprend au moins deux sources lumineuses d'appoint présentant des spectres d'émission distincts. Une telle configuration peut rendre plus aisée l'obtention des conditions d'éclairage souhaitées. Par exemple, le système d'éclairage peut être tel qu'une première de ces deux sources lumineuses d'appoint contribue davantage à l'obtention de la première valeur d'éclairement E1 que l'autre de ces deux sources, et tel que ladite autre de ces deux sources contribue davantage à l'obtention de la deuxième valeur d'éclairement E2 que ladite première de ces deux sources.

Dans certains modes de réalisation, le système d'éclairage peut comprendre un mélangeur de flux lumineux, par exemple un guide de lumière, en entrée duquel sont injectés des flux lumineux respectivement émis par les sources lumineuses d'appoint, et en sortie duquel émerge un faisceau lumineux qui est adapté pour contribuer en tout ou partie à l'éclairement de la zone dentaire prédéterminée avec la première valeur d'éclairement E1 et la deuxième valeur d'éclairement E2. Un tel mélangeur de flux lumineux peut ainsi rendre plus aisé la réunification en un seul et unique faisceau lumineux de plusieurs flux lumineux qui sont émis respectivement par les différentes sources lumineuses d'appoint du système d'éclairage.

Dans certains modes de réalisation, le système d'éclairage peut être configuré pour émettre un faisceau lumineux, par exemple un faisceau qui émerge du mélangeur de flux lumineux précité, qui présente, sur la première plage spectrale, une première valeur d'intensité I1 et, sur la deuxième plage spectrale, une deuxième valeur d'intensité I2, et le système d'éclairage peut être configuré pour que, lorsqu'il fonctionne selon le premier mode d'éclairage, le rapport de la première valeur d'intensité I1 sur la deuxième valeur d'intensité I2 soit inférieur à 13 (treize).

Cette valeur maximale du rapport I1/I2, déterminée au niveau d'une sortie du système d'éclairage, est à mettre en correspondance à la valeur maximale du rapport E1/E2 susmentionnée, déterminée au niveau de la zone dentaire prédéterminée, après propagation de rayons lumineux, en particulier depuis ladite sortie du système d'éclairage. Il est volontairement opté pour une valeur maximale plus grande pour le rapport I1/I2 que pour le rapport E1/E2, afin de tenir compte de l'influence sur les valeurs d'éclairement dans la zone dentaire prédéterminée qu'auraient les éventuels autres faisceaux lumineux, qui seraient obtenus par des moyens autres que les sources lumineuses d'appoint du dispositif et seraient susceptibles d'impacter ladite zone.

Dans certains modes de réalisation, la ou chaque source lumineuse d'appoint peut comprendre une diode électroluminescente, ce qui peut faciliter une mise en œuvre bon marché du dispositif.

Dans certains modes de réalisation, le système d'éclairage peut être tel, lorsqu'il comprend au moins deux diodes électroluminescentes, que chacune de ces deux diodes présente un spectre d'émission distinct l'une de l'autre.

Le système d'éclairage est en outre configuré pour fonctionner selon au moins un deuxième mode d'éclairage distinct du premier, le dispositif comprend un commutateur configuré pour faire commuter le fonctionnement du système d'éclairage entre le premier mode d'éclairage et le deuxième mode d'éclairage. Ainsi, le dispositif d'éclairage dentaire présente une plus grande modularité d'utilisation, le commutateur étant configuré pour, à partir du premier mode d'éclairage, faire commuter le fonctionnement du système d'éclairage dans son deuxième mode d'éclairage, et réciproquement.

Dans certains modes de réalisation, le système d'éclairage peut être configuré pour que le rapport de la première valeur d'éclairement E1 sur la deuxième valeur d'éclairement E2 présente des valeurs différentes dans le premier mode d'éclairage et dans le deuxième mode d'éclairage, respectivement. En d'autres termes, ce rapport E1/E2 peut présenter une première valeur R1, dans le premier mode d'éclairage, et une deuxième valeur R2, dans le deuxième mode d'éclairage, avec R1 différent de R2.

Dans certains modes de réalisation, le système d'éclairage peut être configuré pour que le rapport de la première valeur d'éclairement E1 sur la deuxième valeur d'éclairement E2 soit plus grand dans le premier mode d'éclairage que dans le deuxième mode d'éclairage, ce qui revient à dire que la première valeur R1 précitée est alors supérieure à la deuxième valeur R2 précitée.

Une telle diminution du rapport E1/E2, lorsque l'on passe du premier mode d'éclairage au deuxième mode d'éclairage, peut notamment permettre de tenir compte de l'évolution du contraste entre une portion d'émail à nu (i.e. dépourvue de tartre) et une portion d'émail entartrée en fonction de l'âge de la dent.

En effet, il usuellement observé que l'émail présente une porosité qui s'accentue avec l'âge. Ainsi, lorsque la dent est jeune (i.e. usuellement, lorsque la dent est âgée de moins de 25 ans environ), l'émail est faiblement poreux. Dès lors, une substance révélatrice peut facilement pénétrer à l'intérieur des résidus alimentaires, qui se fixent à une portion d'émail entartrée, mais difficilement pénétrer à l'intérieur d'une portion d'émail dépourvue de tartre. Il en résulte que ladite portion d'émail entartrée fluoresce bien davantage que ladite portion d'émail dépourvue de tartre, de sorte que ces deux portions peuvent être perçues avec un contraste prononcé. En revanche, lorsque la dent vieillit, ce contraste diminue car l'émail devient davantage poreux. En effet, la substance révélatrice peut alors plus facilement pénétrer dans l'émail de la portion dépourvue de tartre. Il en résulte que cette portion dépourvue de tartre fluoresce davantage, ce qui la rend difficilement perceptible par rapport à la portion d'émail entartrée, qui elle continue de fluorescer sensiblement.

Ainsi, le deuxième mode d'éclairage précité peut être mis à profit pour augmenter la deuxième valeur d'éclairement E2 relativement à la première valeur d'éclairement E1, de manière à ce que la zone dentaire soit éclairée avec davantage de longueurs d'onde auxquelles la substance révélatrice n'est pas sensible (i.e. de longueurs d'ondes qui ne sont pas susceptibles d'exciter une émission par fluorescence de cette substance). Il peut ainsi en résulter une augmentation du flux lumineux réfléchi de manière spéculaire sur la dent et, partant l'augmentation de contraste recherchée. En effet, la réflectivité d'une portion d'émail à nu est plus importante que la réflectivité d'une portion entartrée.

Dans certains modes de réalisation, le système d'éclairage peut être configuré pour que le rapport de la première valeur d'éclairement E1 sur la deuxième valeur d'éclairement E2 soit plus petit dans le premier mode d'éclairage que dans le deuxième mode d'éclairage, ce qui revient à dire que la première valeur R1 précitée est alors inférieure à la deuxième valeur R2 précitée.

Une telle augmentation du rapport E1/E2, lorsque l'on passe du premier mode d'éclairage au deuxième mode d'éclairage, peut notamment permettre une amélioration de la visibilité de la fluorescence générée par l'éclairement de la zone dentaire avec la première couleur « dans le bleu ». Ainsi, en passant du premier mode d'éclairage au deuxième mode d'éclairage et réciproquement, le praticien peut ajuster sa perception au besoin et tenir compte des spécificités des dents du patient.

Dans certains modes de réalisation, le système d'éclairage peut être configuré pour que le ratio R2/R1, entre la deuxième valeur R2 du rapport E1/E2 dans le deuxième mode d'éclairage et la première valeur R1 dudit rapport dans le premier mode d'éclairage, soit compris, au choix, entre deux valeurs quelconques parmi toutes les valeurs ci-après : 2 ; 5 ; 10 ; 20. Plus généralement, des essais cliniques complémentaires de la déposante ont permis de vérifier que des performances optimales du dispositif de l'invention sont obtenues lorsque le ratio R2/R1 est compris entre 2 et 20 (bornes supérieure et inférieure incluses), ou entre 0,05 et 0,5 (bornes supérieure et inférieure incluses) en fonction du mode qui est qualifié de « premier » mode d'éclairage ou « deuxième » mode d'éclairage. La valeur de ce ratio R2/R1 pourra être déterminée en fonction des besoins propres du praticien en termes de perception des portions d'émail à nu relativement aux portions d'émail entartrées.

Le certains modes commutateur est configuré pour faire commuter le fonctionnement du système d'éclairage entre le premier mode d'éclairage et le deuxième mode d'éclairage en faisant varier l'une des deux valeurs parmi la première valeur d'éclairement E1 et la deuxième valeur d'éclairement E2, tandis que l'autre de ces deux valeurs demeure inchangée. Cette configuration représente une solution simple pour mettre en œuvre la commutation de fonctionnement précitée. Elle peut également permettre de mieux mettre en évidence les portions d'émail entartrées par rapport aux portions d'émail à nu, en faisant varier uniquement un flux quelconque parmi le flux réfléchi de manière spéculaire sur les dents et le flux émis par fluorescence du révélateur qui a pénétré dans les dents.

Dans certains modes de réalisation, le commutateur peut être configuré pour faire commuter le fonctionnement du système d'éclairage entre le premier mode d'éclairage et le deuxième mode d'éclairage en faisant varier uniquement la deuxième valeur d'éclairement E2, tandis que la première valeur d'éclairement E1 demeure inchangée.

Dans certains modes de réalisation, le commutateur peut être configuré pour faire commuter le fonctionnement du système d'éclairage entre le premier mode d'éclairage et le deuxième mode d'éclairage en faisant varier uniquement la première valeur d'éclairement E1, tandis que la deuxième valeur d'éclairement E2 demeure inchangée.

Dans certains modes de réalisation, le commutateur peut comprendre un actionneur, par exemple un interrupteur, un variateur ou autre, apte à être manœuvré par l'utilisateur pour faire commuter le fonctionnement du système d'éclairage entre le premier mode d'éclairage et le deuxième mode d'éclairage, ce qui peut permettre au praticien de choisir l'instant le plus opportun pour provoquer cette commutation.

Dans certains modes de réalisation, le commutateur peut être configuré pour faire commuter automatiquement (i.e. sans action de la part de l'utilisateur) et répétitivement (par exemple, périodiquement, à une fréquence prédéterminée) le fonctionnement du système d'éclairage entre le premier mode d'éclairage et le deuxième mode d'éclairage.

Ainsi, cette commutation périodique entre les deux modes d'éclairage peut permettre au praticien d'observer avec plus de précision les portions de dents entartrées et les portions de dents dépourvues de tartre par une variation répétitive du contraste entre ces différentes portions provoquée par cette commutation périodique et ce, sans que le praticien n'ait besoin d'intervenir sur le dispositif.

Un deuxième aspect du présent exposé concerne un procédé de configuration d'un éclairage mettant en œuvre un dispositif d'éclairage dentaire selon le premier aspect détaillé ci-avant dans le présent exposé.

Ce procédé comprend une étape de configuration du système d'éclairage, au cours de laquelle on configure le système d'éclairage pour que ce dernier fonctionne selon au moins un premier mode d'éclairage, dans lequel ledit système d'éclairage éclaire une zone dentaire prédéterminée avec, sur une première plage spectrale qui est inférieure à une longueur d'onde de référence comprise entre 405 nm et 475 nm, une première valeur d'éclairement E1 et, sur une deuxième plage spectrale supérieure à cette longueur d'onde de référence, une deuxième valeur d'éclairement E2, et dans lequel le rapport de la première valeur d'éclairement E1 sur la deuxième valeur d'éclairement E2 est inférieur à 12.

Les caractéristiques et avantages précités, ainsi que d'autres, apparaîtront mieux à la lecture de la description détaillée qui suit, d'exemples de réalisation qui sont dépourvus de tout caractère limitatif et qui sont simplement proposés à titre illustratif. Cette description détaillée fait référence aux dessins annexés.

### BREVE DESCRIPTION DES DESSINS

Les dessins annexés sont schématiques et ne sont pas à l'échelle, ils visent avant tout à illustrer les principes mentionnés dans le présent exposé. Sur ces dessins annexés :
- les figures 1A, 1B et 1C montrent respectivement une vue en coupe partielle, une vue de face et une vue en coupe IC-IC d'un premier exemple de réalisation d'un dispositif d'éclairage dentaire conforme au présent exposé ;
- la figure 2 montre le spectre d'émission d'une diode électroluminescente d'une première catégorie utilisée dans ce premier exemple ;
- les figures 3A et 3B montrent différents choix de spectres d'émission pour une diode électroluminescente d'une deuxième catégorie utilisée dans ce premier exemple ;
- la figure 4 montre de manière schématique l'évolution des rapports de valeurs des signaux de commande des diodes précitées en fonction du mode d'éclairage sélectionné avec un deuxième exemple de réalisation d'un dispositif d'éclairage dentaire conforme au présent exposé ;
- les figures 5A, 5B, 5C et 5D montrent des évolutions de contraste entre portions d'émail à nu et portions d'émail entartrées, qui sont obtenues avec le dispositif selon le deuxième exemple précité, en fonction du mode d'éclairage sélectionné ;
- la figure 6A montre de manière schématique l'éclairage d'une zone dentaire prédéterminée par le dispositif selon le premier ou le deuxième exemple de réalisation précité ;
- la figure 6B montre de manière schématique l'éclairage d'une zone dentaire prédéterminée par un dispositif d'éclairage dentaire selon un troisième exemple de réalisation conforme au présent exposé ;
- la figure 7 est un graphique illustrant de manière schématique un exemple de détermination de la première valeur d'éclairement E1 et de la deuxième valeur d'éclairement E2 selon l'un quelconque des trois exemples de réalisation précités.

### DESCRIPTION DETAILLEE D'EXEMPLES DE REALISATION

### Premier exemple de réalisation

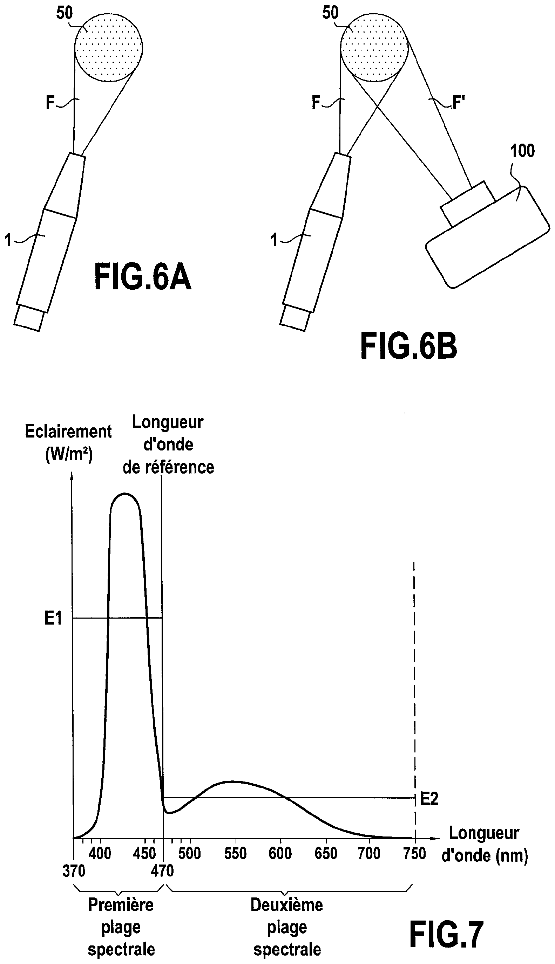

Comme illustré à la figure 6A, un dispositif d'éclairage dentaire selon ce premier exemple comprend un système d'éclairage configuré pour éclairer une zone dentaire 50 prédéterminée.

Dans le cadre du présent exposé, on entend désigner par l'expression « une zone dentaire prédéterminée », une surface située dans un plan imaginaire dont les dimensions caractéristiques sont de l'ordre de grandeur de celles de la partie anatomique qui est usuellement observée par un praticien lors d'un acte de dentisterie.

Par exemple, cette surface peut présenter des dimensions caractéristiques comprises entre 0,5 centimètre et 5 centimètres, par exemple entre 1 centimètre et 3 centimètres.

Sur la figure 6A, on a arbitrairement illustré, de manière non limitative, cette surface par un disque, dont le diamètre est égal à une dimension caractéristique du type de celles précédemment décrites. On pourrait toutefois prévoir, sans sortir du cadre du présent exposé, une toute autre forme pour cette surface (par exemple un rectangle ou un carré dont les côtés sont égaux à des dimensions caractéristiques du type de celles précédemment décrites), pourvu seulement que ses dimensions caractéristiques soient de l'ordre de grandeur précité.

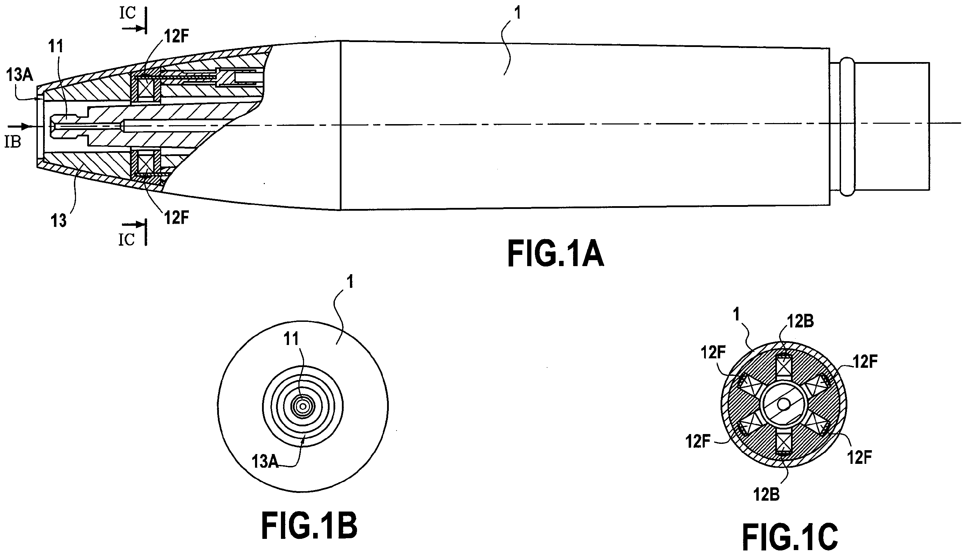

Selon cet exemple, le dispositif d'éclairage dentaire comprend un instrument d'appoint 1, mieux visible sur les figures 1A à 1C, et le système d'éclairage comprend au moins une source lumineuse d'appoint solidaire de cet instrument 1.

Selon cet exemple, le système d'éclairage de cet instrument 1 est configuré pour émettre un premier flux lumineux F qui, après propagation, éclaire la zone dentaire 50.

En particulier, comme illustré à la figure 7, le système d'éclairage de cet instrument 1 est configuré pour éclairer la zone dentaire 50 avec, sur une première plage spectrale qui est inférieure à une longueur d'onde de référence comprise entre 405 nm et 475 nm, une première valeur d'éclairement E1 et, sur une deuxième plage spectrale supérieure à cette longueur d'onde de référence, une deuxième valeur d'éclairement E2.

Comme déjà indiqué précédemment, on entend dans ce document par « valeur d'éclairement » ou, plus précisément, par « valeur moyenne d'éclairement », la définition radiométrique conventionnelle de ce terme, à savoir dans le cas présent la puissance lumineuse moyenne reçue par unité de surface, cette valeur étant calculée par intégration sur l'ensemble de la plage spectrale considérée (i.e. la première ou la deuxième plage spectrale dans le cadre de l'invention).

Plus particulièrement, selon cet exemple, la longueur d'onde de référence est choisie égale à 470 nm ; la première plage spectrale est comprise entre une borne inférieure, qui égale à 370 nm, et cette longueur d'onde de référence à 470 nm ; et la deuxième plage spectrale est comprise entre cette longueur d'onde de référence à 470 nm et une borne supérieure, qui est égale à 750 nm.

Par ailleurs, selon cet exemple, le système d'éclairage est configuré pour émettre une première pluralité de rayons lumineux, avec un premier spectre continu, qui plus est sur l'étendue entière de la première plage spectrale. Ainsi, chaque rayon lumineux qui appartient à cette première pluralité est émis par le système d'éclairage à une longueur d'onde qui est incluse dans la première plage spectrale, en particulier comprise entre 370 nm et 470 nm.

On pourrait toutefois prévoir, sans sortir du cadre du présent exposé, que le système d'éclairage soit configuré pour émettre cette première pluralité de rayons lumineux avec un spectre discontinu et/ou ne s'étalant pas sur l'étendue entière de la première plage spectrale (en émettant, par exemple, une ou plusieurs bandes ou raies espacées les unes des autres, chacune de ces bandes ou raies ayant une longueur d'onde centrale incluse dans la première plage spectrale).

Par ailleurs, les rayons lumineux de la première pluralité ainsi émis éclairent ensemble, après propagation, la zone dentaire 50 avec une première valeur d'éclairement E1.

Selon cet exemple, cette première valeur d'éclairement E1 est calculée de la manière suivante :
- pour chaque longueur d'onde L1 appartenant à la première plage spectrale, on calcule l'éclairement élémentaire dE1(L1) de la manière suivante :
   ∘ pour chaque point de coordonnées (x, y) appartenant à la surface S définissant la zone dentaire 50, on calcule l'éclairement élémentaire d²E1(x, y, L1) ;
   ∘ on calcule, pour déterminer dE1(L1), la valeur moyenne de cet éclairement élémentaire d²E1 (x, y , L1) sur toute la surface S ;
- on calcule, pour déterminer la valeur E1, la valeur moyenne de cet éclairement élémentaire dE1(L1) sur toute la première plage spectrale.

On obtient ainsi la première valeur moyenne d'éclairement E1 qui équivaut à la puissance lumineuse moyenne reçue par unité de surface, cette valeur moyenne E1 étant calculée par intégration sur la totalité de la première plage spectrale.

En outre, selon cet exemple, le système d'éclairage est configuré pour émettre une deuxième pluralité de rayons lumineux, avec un deuxième spectre continu, qui plus est sur l'étendue entière de la deuxième plage spectrale. Ainsi, chaque rayon lumineux qui appartient à cette deuxième pluralité est émis par le système d'éclairage à une longueur d'onde qui est incluse dans la deuxième plage spectrale, en particulier comprise entre 470 nm et 750 nm.

On pourrait toutefois prévoir, sans sortir du cadre du présent exposé, que le système d'éclairage soit configuré pour émettre cette deuxième pluralité de rayons lumineux avec un spectre discontinu et/ou ne s'étalant pas sur l'étendue entière de la deuxième plage spectrale (en émettant, par exemple, une ou plusieurs bandes ou raies espacées les unes des autres, chacune de ces bandes ou raies ayant une longueur d'onde centrale incluse dans la deuxième plage spectrale).

Par ailleurs, les rayons lumineux de la deuxième pluralité ainsi émis éclairent ensemble, après propagation, la zone dentaire 50 avec une deuxième valeur d'éclairement E2.

Selon cet exemple, cette deuxième valeur d'éclairement E2 est calculée de la manière suivante :
- pour chaque longueur d'onde L2 appartenant à la deuxième plage spectrale, on calcule l'éclairement élémentaire dE2(L2) de la manière suivante :
   ∘ pour chaque point de coordonnées (x, y) appartenant à la surface S définissant la zone dentaire 50, on calcule l'éclairement élémentaire d²E2(x, y, L2) ;
   ∘ on calcule, pour déterminer dE2(L2), la valeur moyenne de cet éclairement élémentaire d²E2 (x, y , L2) sur toute la surface S ;
- on calcule, pour déterminer la valeur E2, la valeur moyenne de cet éclairement élémentaire dE2(L2) sur toute la deuxième plage spectrale.

On obtient ainsi la deuxième valeur moyenne d'éclairement E2 qui équivaut à la puissance lumineuse moyenne reçue par unité de surface, cette valeur moyenne E2 étant calculée par intégration sur la totalité de la deuxième plage spectrale.

Selon cet exemple, le système d'éclairage est configuré pour fonctionner seulement selon un premier et unique mode d'éclairage, dans lequel le rapport de la première valeur d'éclairement E1 sur la deuxième valeur d'éclairement E2 est inférieur à une valeur plafond prédéterminée.

Dans cet exemple, ce rapport E1/E2 dans ce premier mode d'éclairage est choisi égal à environ 8 (huit).

On va à présent décrire plus en détail l'instrument d'appoint 1 qui comprend ce système d'éclairage, à l'aide des figures 1A à 1C.

Selon cet exemple, cet instrument d'appoint comporte un corps présentant une partie de fixation 11 apte à coopérer avec un outil (non représenté, cet outil peut, par exemple, être un outil de détartrage conventionnel) pour fixer ledit outil et le corps l'un par rapport à l'autre.

Selon cet exemple, la partie de fixation 11 correspond à une partie d'extrémité du corps 1, et présente des dimensions adaptées pour permettre à cette partie d'extrémité d'être introduite dans la bouche d'un patient pour procéder à un acte de dentisterie à l'aide de l'outil à fixer sur ledit corps 1.

Par ailleurs, le système d'éclairage comprend une pluralité de sources lumineuses d'appoint, dissociées les unes des autres.

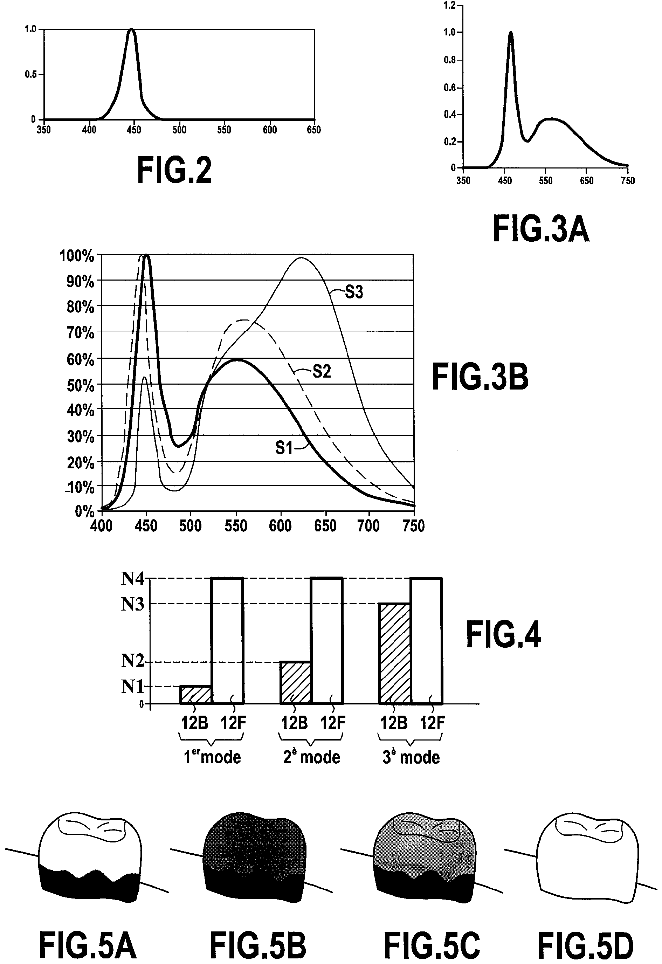

Selon cet exemple, le système d'éclairage comprend un premier groupe constitué d'une ou plusieurs sources lumineuses d'appoint, qui présentent, chacune, un seul et même premier spectre d'émission identique (mieux visible sur la figure 2) ; et au moins un deuxième groupe constitué d'une ou plusieurs autres sources lumineuses d'appoint, qui présentent, chacune, un seul et même deuxième spectre d'émission, différent du premier spectre d'émission (mieux visible sur les figures 3A et 3B, qui présentent différentes variantes de ce deuxième spectre).

En particulier, dans cet exemple, le premier groupe précité est constitué de quatre sources lumineuses d'appoint, tandis que le deuxième groupe précité est constitué de deux sources lumineuses d'appoint. Ainsi, dans cet exemple, le système d'éclairage comprend, un total de six sources lumineuses d'appoint.

Plus particulièrement, chacune de ces six sources lumineuses d'appoint comprend une diode électroluminescente. Ainsi, dans cet exemple, le premier groupe précité est constitué de quatre diodes électroluminescentes 12F, qui, chacune, présentent le premier spectre d'émission précité. D'un autre côté, le deuxième groupe précité est constitué de deux diodes électroluminescentes 12B, qui, chacune, présente le deuxième spectre d'émission précité.

Par ailleurs, selon cet exemple, l'instrument d'appoint présente une pluralité de logements, dans laquelle est respectivement montée la pluralité de sources lumineuses d'appoint 12F, 12B. Dans cet exemple, ces logements sont disposés à intervalles sensiblement réguliers angulairement (voir la figure 1C), au voisinage de la partie de fixation 11, en particulier légèrement en retrait de l'extrémité depuis laquelle l'outil est destiné à faire saillie depuis le corps 1.

En outre, selon cet exemple, le système d'éclairage comprend un mélangeur de flux lumineux, en particulier un guide de lumière 13, en entrée duquel sont injectés des flux lumineux respectivement émis par chacune des diodes 12F, 12B, et en sortie 13A duquel émerge un faisceau lumineux F (mieux visible sur la figure 6A) adapté pour, à lui seul, permettre l'éclairement de la zone dentaire 50 avec les première et deuxième valeurs d'éclairement E1 et E2 désirées.

En particulier, ce guide de lumière 13, visible de face sur la figure 1B, est conformé, au moins en sortie, en une couronne lumineuse 13A. Il permet de conduire la lumière des diodes 12F, 12B jusqu'à l'extrémité du corps 1 depuis laquelle l'outil est destiné à faire saillie.

Par ailleurs, on a représenté sur la figure 2 le premier spectre d'émission de chacune des diodes 12F du premier groupe précité.

Selon cet exemple, ce premier spectre est constitué d'un unique pic de largeur à mi-hauteur d'environ 20nm et centré sur une longueur d'onde d'environ 450nm. Ainsi, chaque diode 12F du premier groupe émet des rayons lumineux uniquement dans la première plage spectrale définie à la figure 7.

Dès lors, chaque diode 12F participe uniquement à la réalisation d'au moins une partie de l'éclairage « dans le bleu » de la zone dentaire 50 en vue de l'obtention de la première valeur d'éclairement E1.Inversement, ces diodes 12F n'apportent aucune contribution pour l'obtention de l'éclairage dans une couleur « plus chaude que le bleu » de cette zone 50 avec la deuxième valeur d'éclairement E2.

Par ailleurs, on a représenté sur la figure 3A une première variante S1 du deuxième spectre d'émission de chacune des diodes 12B du deuxième groupe précité.

En particulier, ce deuxième spectre selon cette première variante comprend un premier pic de plus grande intensité, et un deuxième pic de plus faible intensité.

Plus particulièrement, ce premier pic est centré sur une première longueur d'onde plus petite qu'une deuxième longueur d'onde sur laquelle est centré le deuxième pic précité. Notamment, ces première et deuxième longueurs d'onde sont respectivement égales à environ 455 nm et environ 560 nm.

En outre, ce premier pic présente une largeur à mi-hauteur plus petite que celle du deuxième pic (notamment environ 20 nm contre environ 120 nm).

Dès lors, chaque diode 12B participe activement non seulement à la réalisation d'au moins une partie de l'éclairage « dans le bleu » de la zone dentaire 50 en vue de l'obtention de la première valeur d'éclairement E1, mais participe également activement à la réalisation d'au moins une partie de l'éclairage dans une couleur « plus chaude que le bleu » de cette zone 50 en vue de l'obtention de la deuxième valeur d'éclairement E2.

Par ailleurs, la température de couleur associée à ce deuxième spectre d'émission selon cette première variante S1 est d'environ 6500 K.

En outre, on a représenté à la figure 3B, en plus de la première variante S1 précitée, des deuxième et troisième variantes S2 et S3 du deuxième spectre d'émission des diodes 12B du deuxième groupe.

Ces deuxième et troisième variantes S2 et S3 sont analogues à la première variante S1, de sorte que leur description ne sera pas reprise en détail. Elles présentent néanmoins les quelques différences ci-après évoquées :
La deuxième variante S2, et encore plus la troisième variante S3, contribuent davantage à l'obtention de la deuxième valeur d'éclairement E2, leurs deuxièmes pics respectifs étant plus prononcés que le deuxième pic de la première variante S1.

Par ailleurs, la troisième variante S3 participe sensiblement moins à l'obtention de la première valeur d'éclairement E1, son premier pic étant amoindri par rapport aux premiers pics respectifs de la première variante S1 et de la deuxième variante S2, sensiblement égaux.

Enfin, la température de couleur associée à la deuxième variante S2 est d'environ 4500K, tandis que celle associée à la troisième variante S3 est d'environ 3000 K.

Par ailleurs, selon cet exemple, le dispositif d'éclairage dentaire comprend un système de commande (non représenté) couplé au système d'éclairage et configuré pour commander le fonctionnement dudit système d'éclairage selon le premier mode d'éclairage précité.

En particulier, le système de commande est configuré pour générer une pluralité de signaux de commande qui sont respectivement transmis à la pluralité de diodes 12B, 12F du système d'éclairage pour commander leur émission lumineuse. Notamment, dans cet unique premier mode d'éclairage, ces signaux de commande adoptent des premières valeurs de signal respectives qui sont adaptées pour permettre au système d'éclairage d'éclairer la zone dentaire 50 avec le rapport E1/E2 égal, dans cet exemple, à environ 8 comme précédemment indiqué.

### Deuxième exemple de réalisation

A l'aide des figures 4 à 5D, on va à présent décrire un dispositif d'éclairage dentaire selon un deuxième exemple de réalisation conforme au présent exposé.

Un dispositif d'éclairage dentaire selon ce deuxième exemple de réalisation diffère du dispositif d'éclairage dentaire selon le premier exemple de réalisation précédemment décrit, uniquement en ce que le système d'éclairage du dispositif d'éclairage dentaire selon ce deuxième exemple est configuré pour fonctionner selon non seulement le premier mode d'éclairage décrit en association avec le premier exemple, mais également selon au moins un deuxième mode d'éclairage distinct de ce premier mode. En effet, comme indiqué lors de la description du premier exemple de réalisation, le système d'éclairage du dispositif d'éclairage dentaire selon ce premier exemple est configuré pour fonctionner uniquement selon le premier mode d'éclairage.

En particulier, selon ce deuxième exemple, le système d'éclairage est configuré pour fonctionner selon un mode quelconque parmi :
- le premier mode d'éclairage précédemment décrit en association avec le premier exemple. Ce premier mode d'éclairage permet d'obtenir une première valeur R1 pour le rapport E1/E2 ;
- un deuxième mode d'éclairage, distinct de ce premier mode, qui permet d'obtenir une deuxième valeur R2 pour le rapport E1/E2 qui est inférieure à R1 ;
- et un troisième mode d'éclairage, distinct de ce premier mode et de ce deuxième mode, qui permet d'obtenir une troisième valeur R3 pour le rapport E1/E2 qui est inférieure à R2 ;

A noter que dans une alternative à ce deuxième exemple, le système d'éclairage pourrait être configuré pour commuter seulement entre les premier et deuxième modes d'éclairage mentionnés ci-dessus.

Selon ce deuxième exemple, le passage d'un mode d'éclairage à un autre s'effectue à l'aide d'un commutateur (non représenté), qui est configuré pour faire commuter le fonctionnement du système d'éclairage d'un mode à un autre.

En particulier, le commutateur d'un dispositif selon ce deuxième exemple est couplé au système de commande (lequel est décrit plus en détail en association avec le premier exemple de réalisation précité), et est configuré pour faire commuter le système de commande d'un mode d'éclairage à un autre.

Par ailleurs, le commutateur est configuré pour provoquer des variations des valeurs respectives de tout ou partie des signaux de commande générés par le système de commande, de manière à faire varier d'un mode d'éclairage à un autre le rapport E1/E2 entre les trois valeurs R1, R2 et R3 précitées.

En particulier, comme illustré à la figure 4, dans le premier mode d'éclairage, les signaux de commande respectivement associés aux diodes 12B du deuxième groupe de diodes adoptent, chacun, une valeur N1, tandis que les signaux de commande respectivement associés aux diodes 12F du premier groupe de diodes adoptent, chacun, une valeur N4.

En outre, dans le deuxième mode d'éclairage, les signaux de commande respectivement associés aux diodes 12B du deuxième groupe de diodes adoptent, chacun, une valeur N2 supérieure à N1, tandis que les signaux de commande respectivement associés aux diodes 12F du premier groupe de diodes adoptent, chacun, la valeur N4 précitée.

Enfin, dans le troisième mode d'éclairage, les signaux de commande respectivement associés aux diodes 12B du deuxième groupe de diodes adoptent, chacun, une valeur N3 supérieure à N2, tandis que les signaux de commande respectivement associés aux diodes 12F du premier groupe de diodes adoptent, chacun, la valeur N4 précitée.

Dès lors, le fait que la valeur N3 soit supérieure à la valeur N2, qui elle-même est supérieure à N1, tandis que la valeur N4 demeure inchangée pour les trois modes d'éclairage, font que la première valeur R1 est supérieure à la deuxième valeur R2, qui elle-même est supérieure à la troisième valeur R3.

Par ailleurs, dans cet exemple, les signaux de commande respectivement associés aux diodes 12F du premier groupe de diodes peuvent être des signaux binaires (en tout ou rien), puisqu'il n'est pas nécessaire qu'ils génèrent d'autres valeurs non nulles que la valeur N4 précitée.

D'un autre côté, dans cet exemple, les signaux de commande respectivement associés aux diodes 12B du deuxième groupe de diodes peuvent être des signaux analogiques ou numériques adaptés pour générer séquentiellement au moins les trois valeurs non nulles N1, N2 et N3 précitées.

A titre d'exemple illustratif et non limitatif, on choisit arbitrairement R1 égal à 8, R2 égal à 5 et R3 égal à 1,5. On observe alors les contrastes illustrés aux figures 5A à 5D ci-après décrites. Comme indiqué précédemment, on obtient des conditions d'observation optimales lorsque le ratio entre les rapports E1/E2 pour chaque commutation de mode d'éclairement est compris entre 2 et 20 (bornes incluses) ou, le cas échéant, entre 0,05 et 0,5 (bornes incluses). En particulier, la figure 5A représente la perception que peut avoir un praticien d'une première dent, dont l'émail est faiblement poreux, lorsque cette dent est éclairée sous le premier mode d'éclairage, avec la première valeur R1. Dans ce cas, le contraste entre portions de la dent entartrées et portions de la dent dépourvues de tartre est bon car la substance révélatrice peut difficilement pénétrer dans ces portions dépourvues de tartre, de sorte que le flux réfléchi de manière spéculaire par ces portions dépourvues de tartre est prépondérant sur le flux qu'elles émettent pas fluorescence de la substance révélatrice, et ce, même si la dent est éclairée avec une deuxième valeur d'éclairement E2 faible par rapport à la première valeur d'éclairement E1. Dès lors, on peut qualifier le premier mode d'éclairage de mode adapté pour une observation du tartre pour un émail faiblement poreux.

Par ailleurs, la figure 5B représente la perception que peut avoir un praticien d'une deuxième dent, dont l'émail est davantage poreux que celui de la première dent précitée, lorsque cette deuxième dent est éclairée également sous le premier mode d'éclairage, avec la première valeur R1. Dans ce cas, le contraste entre portions de la dent entartrées et portions de la dent dépourvues de tartre est médiocre car la substance révélatrice peut aisément pénétrer dans ces portions dépourvues de tartre. Il en résulte que la deuxième valeur d'éclairement E2 est cette fois insuffisante par rapport à la première valeur d'éclairement E1 pour permettre au flux réfléchi de manière spéculaire par la surface de l'émail de ces portions dépourvues de tartre d'être sensiblement plus important que le flux émis par fluorescence depuis l'intérieur poreux de ces portions. Le praticien perçoit alors une couleur jaunâtre pour la dent dans toute son intégralité.

Pour remédier à ce problème, on peut observer cette deuxième dent sous le deuxième mode d'éclairage, dans lequel le flux réfléchi de manière spéculaire est augmenté par rapport au flux émis par fluorescence, grâce au fait que la deuxième valeur R2 du rapport E1/E2 est inférieure à la première valeur R1, sans pour autant masquer l'éclairage « dans le bleu » qui résulte de la première valeur d'éclairage E1. Dès lors, le praticien peut observer cette deuxième dent avec le contraste illustré à la figure 5C. On peut ainsi qualifier le deuxième mode d'éclairage de mode adapté pour une observation du tartre pour un émail sensiblement poreux.

Enfin, la figure 5D représente la perception que peut avoir un praticien de la première dent ou de la deuxième dent précitées, lorsqu'elles sont éclairées sous le troisième mode d'éclairage. Dans ce troisième mode, le flux réfléchi de manière spéculaire est encore davantage augmenté par rapport au flux émis par fluorescence, grâce au fait que la troisième valeur R3 du rapport E1/E2 est inférieure à la deuxième valeur R2. Mais cette fois la deuxième valeur d'éclairage E2 est trop importante par rapport à la première valeur d'éclairage E1, de sorte que l'éclairage dans la couleur « plus chaude que le bleu » masque sensiblement l'éclairage « dans le bleu ». Il en résulte que, dans ce troisième mode d'éclairage, le contraste est mauvais, seule la structure anatomique de la dent pouvant être observée par le praticien grâce aux réflexions spéculaires depuis la surface de l'émail qu'il perçoit. On peut ainsi qualifier le troisième mode d'éclairage de mode adapté pour une observation de la dent sans mise en évidence du tartre.

Il indiqué que toutes les autres caractéristiques décrites en association avec le premier exemple de réalisation, qui n'ont pas été à nouveau décrites en association avec le deuxième exemple de réalisation dans un souci de rationalisation de la taille du présent exposé, peuvent bien évidemment être reprises, au choix, seules ou en combinaison, par ce deuxième exemple de réalisation.

### Troisième exemple de réalisation

A la figure 6B, on a représenté un dispositif d'éclairage dentaire selon un troisième exemple de réalisation conforme au présent exposé.

Un dispositif d'éclairage dentaire selon ce troisième exemple de réalisation diffère des dispositifs d'éclairage dentaires selon les premier et deuxième exemples de réalisation précédemment décrits, uniquement en ce que le système d'éclairage du dispositif d'éclairage dentaire selon le troisième exemple comprend non seulement un instrument d'appoint 1, qui intègre une pluralité de sources lumineuses d'appoint configurées pour éclairer la surface de la zone dentaire 50 avec un premier flux lumineux F, mais également un scialytique 100, qui intègre au moins une source lumineuse d'ambiance configurée pour éclairer la surface de la zone dentaire 50 avec un deuxième flux F'.

Ainsi, dans ce troisième exemple, la source lumineuse d'ambiance du scialytique peut participer activement à l'obtention de la valeur du rapport E1/E2 souhaitée, tandis que, dans les premier et deuxième exemples de réalisation, les sources lumineuses d'appoint permettaient à elles toutes seules d'obtenir ladite valeur souhaitée du rapport E1/E2.

Il indiqué que toutes les autres caractéristiques décrites en association avec le premier exemple de réalisation ou le deuxième exemple de réalisation, qui n'ont pas été à nouveau décrites en association avec le troisième exemple de réalisation dans un souci de rationalisation de la taille du présent exposé, peuvent bien évidemment être reprises, au choix, seules ou en combinaison, par ce troisième exemple de réalisation.

En outre, il est rappelé que les modes ou exemples de réalisation décrits dans le présent exposé sont donnés à titre illustratif et non limitatif, une personne du métier pouvant facilement, au vu de cet exposé, modifier ces modes ou exemples de réalisation, ou en envisager d'autres, tout en restant dans la portée de l'invention.

De plus, les différentes caractéristiques de ces modes ou exemples de réalisation peuvent être utilisées seules ou être combinées entre elles. Lorsqu'elles sont combinées, ces caractéristiques peuvent l'être comme décrit ci-dessus ou différemment, l'invention ne se limitant pas aux combinaisons spécifiques décrites dans le présent exposé. En particulier, sauf précision contraire, une caractéristique décrite en relation avec un mode ou exemple de réalisation peut être appliquée de manière analogue à un autre mode ou exemple de réalisation.

## Revendications

1. Dispositif d'éclairage comprenant au moins un système d'éclairage (1, 100) comportant :
un instrument d'appoint (1),
au moins une première source lumineuse d'appoint (12F) solidaire de l'instrument d'appoint (1) et configurée pour éclairer une zone dentaire (50) prédéterminée, sur une première plage spectrale comprise entre 370 nm et une longueur d'onde de référence, la longueur d'onde de référence étant comprise entre 405 nm et 475 nm, avec une première valeur moyenne d'éclairement E1, et
au moins une seconde source lumineuse d'appoint (12B) solidaire de l'instrument d'appoint (1) configurée pour éclairer la zone dentaire (50) prédéterminée, sur une deuxième plage spectrale comprise entre cette longueur d'onde de référence et 780 nm, avec une deuxième valeur moyenne d'éclairement E2,
le système d'éclairage (1, 100) étant configuré pour fonctionner selon au moins un premier mode d'éclairage, dans lequel le rapport de la première valeur moyenne d'éclairement E1 sur la deuxième valeur moyenne d'éclairement E2 est supérieur à 1 et inférieur à 12, et
le système d'éclairage étant en outre configuré pour fonctionner selon au moins un deuxième mode d'éclairage distinct du premier, le dispositif comprenant un système de commande des première et seconde sources lumineuses et un commutateur couplé au système de commande et configuré pour commander le système de commande pour faire commuter le fonctionnement du système d'éclairage entre le premier mode d'éclairage et le deuxième mode d'éclairage pour ajuster la perception d'un utilisateur du dispositif au besoin et tenir compte des spécificités des dents éclairées, la commutation étant réalisée en faisant varier l'une des deux valeurs parmi la première valeur moyenne d'éclairement E1 et la deuxième valeur moyenne d'éclairement E2, tandis que l'autre de ces deux valeurs demeure inchangée, et le système d'éclairage étant configuré pour que le rapport de la première valeur moyenne d'éclairement E1 sur la deuxième valeur moyenne d'éclairement E2 présente des valeurs différentes dans le premier mode d'éclairage et dans le deuxième mode d'éclairage, respectivement.

2. Dispositif selon la revendication 1, dans lequel le système d'éclairage (1, 100) est configuré pour, dans le premier mode d'éclairage, éclairer la zone dentaire (50) prédéterminée avec la première valeur moyenne d'éclairement E1 qui est supérieure à 1 W/m².

3. Dispositif selon l'une quelconque des revendications 1 ou 2, dans lequel, dans le premier mode d'éclairage, ledit rapport est inférieur à 10.

4. Dispositif selon l'une quelconque des revendications 1 à 3, dans lequel la longueur d'onde de référence est comprise entre 440 nm et 460 nm.

5. Dispositif selon l'une quelconque des revendications 1 à 4, comprenant un scialytique (100), et dans lequel le système d'éclairement comprend au moins une source lumineuse d'ambiance solidaire de ce scialytique.

6. Dispositif selon la revendication 5, dans lequel l'instrument d'appoint (1) comporte un corps présentant une partie de fixation (11) apte à coopérer avec un outil, par exemple un détartreur, pour fixer ledit outil et le corps l'un par rapport à l'autre, et au moins un logement disposé au voisinage de la partie de fixation (11) et dans lequel est montée ladite au moins une source lumineuse d'appoint (12B, 12F).

7. Dispositif selon la revendication 5 ou 6, dans lequel les sources lumineuses d'appoint (12B, 12F) sont discrètes.

8. Dispositif selon la revendication 7, dans lequel les sources lumineuses d'appoint (12B, 12F) présentent des spectres d'émission distincts.

9. Dispositif selon la revendication 7 ou 8, dans lequel le système d'éclairage comprend un mélangeur de flux lumineux (13), par exemple un guide de lumière, en entrée duquel sont injectés des flux lumineux respectivement émis par les sources lumineuses d'appoint (12B, 12F), et en sortie duquel émerge un faisceau lumineux (F) adapté pour contribuer en tout ou partie à l'éclairement de la zone dentaire (50) prédéterminée avec la première valeur moyenne d'éclairement E1 et la deuxième valeur moyenne d'éclairement E2.

10. Dispositif selon l'une quelconque des revendications 6 à 9, dans lequel la ou chaque source lumineuse d'appoint (12B, 12F) comprend une diode électroluminescente.

11. Procédé de configuration d'un éclairage mettant en œuvre un dispositif d'éclairage dentaire selon l'une quelconque des revendications 1 à 10, le procédé comprenant une étape de configuration du système d'éclairage (1, 100), au cours de laquelle on configure le système d'éclairage pour que ce dernier fonctionne selon au moins un premier mode d'éclairage, dans lequel ledit système d'éclairage éclaire une zone dentaire (50) prédéterminée, sur une première plage spectrale comprise entre 370 nm et une longueur d'onde de référence, la longueur d'onde de référence étant comprise entre 405 nm et 475 nm, avec une première valeur moyenne d'éclairement E1 et, sur une deuxième plage spectrale comprise entre cette longueur d'onde de référence et 780 nm, avec une deuxième valeur moyenne d'éclairement E2, et dans lequel le rapport de la première valeur moyenne d'éclairement E1 sur la deuxième valeur moyenne d'éclairement E2 est supérieur à 1 et inférieur à 12, le système d'éclairage étant en outre configuré pour fonctionner selon au moins un deuxième mode d'éclairage distinct du premier, le dispositif comprenant un commutateur configuré pour faire commuter le fonctionnement du système d'éclairage entre le premier mode d'éclairage et le deuxième mode d'éclairage pour ajuster la perception d'un utilisateur du dispositif au besoin et tenir compte des spécificités des dents éclairées, la commutation étant réalisée en faisant varier l'une des deux valeurs parmi la première valeur moyenne d'éclairement E1 et la deuxième valeur moyenne d'éclairement E2, tandis que l'autre de ces deux valeurs demeure inchangée, et le système d'éclairage étant configuré pour que le rapport de la première valeur moyenne d'éclairement E1 sur la deuxième valeur moyenne d'éclairement E2 présente des valeurs différentes dans le premier mode d'éclairage et dans le deuxième mode d'éclairage, respectivement.

## Patentansprüche

1. Beleuchtungseinrichtung, die mindestens ein Beleuchtungssystem (1, 100) umfasst, das beinhaltet:
ein Zusatzinstrument (1),
mindestens eine erste Zusatzlichtquelle (12F), die fest mit dem Zusatzinstrument (1) verbunden ist und dazu ausgestaltet ist, eine vorbestimmte Zahnzone (50) auf einem ersten Spektralbereich, der zwischen 370 nm und einer Bezugswellenlänge beträgt, wobei die Bezugswellenlänge zwischen 405 nm und 475 nm beträgt, mit einem ersten Beleuchtungsmittelwert E1 zu beleuchten, und
mindestens eine zweite fest mit dem Zusatzinstrument (1) verbundene Zusatzlichtquelle (12B), die dazu ausgestaltet ist, die vorbestimmte Zahnzone (50) auf einem zweiten Spektralbereich, der zwischen dieser Bezugswellenlänge und 780 nm beträgt, mit einem zweiten Beleuchtungsmittelwert E2 zu beleuchten,
wobei das Beleuchtungssystem (1, 100) dazu ausgestaltet ist, gemäß mindestens einem ersten Beleuchtungsmodus betrieben zu werden, in dem das Verhältnis des ersten Beleuchtungsmittelwerts E1 zu dem zweiten Beleuchtungsmittelwert E2 größer als 1 und keiner als 12 ist, und
das Beleuchtungssystem ferner dazu ausgestaltet ist, gemäß mindestens einem zweiten Beleuchtungsmodus betrieben zu werden, der sich von dem ersten unterscheidet, wobei die Vorrichtung ein System zu Steuerung der ersten und der zweiten Lichtquelle und einen Schalter umfasst, der an das Steuersystem gekoppelt ist und dazu ausgestaltet ist, das Steuersystem zu steuern, um den Betrieb des Beleuchtungssystems zwischen dem ersten Beleuchtungsmodus und dem zweiten Beleuchtungsmodus zu schalten, um die Wahrnehmung eines Benutzers der Vorrichtung nach Bedarf anzupassen und die Besonderheiten der beleuchteten Zähne zu berücksichtigen, wobei das Schalten ausgeführt wird, indem der eine der zwei Werte von dem ersten Beleuchtungsmittelwert E1 und dem zweiten Beleuchtungsmittelwert E2 verändert wird, während der andere dieser zwei Werte unverändert bleibt, und das Beleuchtungssystem so ausgestaltet ist, dass das Verhältnis des ersten Beleuchtungsmittelwerts E1 zu dem zweiten Beleuchtungsmittelwert E2 in dem ersten Beleuchtungsmodus beziehungsweise in dem zweiten Beleuchtungsmodus unterschiedliche Werte aufweist.

2. Vorrichtung nach Anspruch 1, wobei das Beleuchtungssystem (1, 100) dazu ausgestaltet ist, in dem ersten Beleuchtungsmodus die vorbestimmte Zahnzone (50) mit dem ersten Beleuchtungsmittelwert E1 zu beleuchten, der höher als 1 W/m² ist.

3. Vorrichtung nach einem der Ansprüche 1 oder 2, wobei das Verhältnis in dem ersten Beleuchtungsmodus kleiner als 10 ist.

4. Vorrichtung nach einem der Ansprüche 1 bis 3, wobei die Bezugswellenlänge zwischen 440 nm und 460 nm beträgt.

5. Vorrichtung nach einem der Ansprüche 1 bis 4, die eine Operationsleuchte (100) umfasst, und wobei das Beleuchtungssystem mindestens eine Umgebungslichtquelle umfasst, die fest mit dieser Operationsleuchte verbunden ist.

6. Vorrichtung nach Anspruch 5, wobei das Zusatzinstrument (1) einen Körper, der einen Befestigungsteil (11) aufweist, der dazu geeignet ist, mit einem Werkzeug, zum Beispiel einem Zahnsteinentferner, zusammenzuwirken, um das Werkzeug und den Körper aneinander zu befestigen, und mindestens eine Aufnahme beinhaltet, die in der Nachbarschaft des Befestigungsteils (11) angeordnet ist und in der die mindestens eine Zusatzlichtquelle (12B, 12F) montiert ist.

7. Vorrichtung nach Anspruch 5 oder 6, wobei die Zusatzlichtquellen (12B, 12F) getrennt sind.

8. Vorrichtung nach Anspruch 7, wobei die Zusatzlichtquellen (12B, 12F) unterschiedliche Emissionsspektren aufweisen.

9. Vorrichtung nach Anspruch 7 oder 8, wobei das Beleuchtungssystem einen Lichtstrommischer (13), zum Beispiel einen Lichtleiter, umfasst, an dessen Eingang Lichtströme eingekoppelt werden, die jeweils von den Zusatzlichtquellen (12B, 12F) emittiert werden, und an dessen Ausgang ein Lichtstrahl (F) austritt, der dazu geeignet ist, vollständig oder teilweise zur Beleuchtung der vorbestimmten Zahnzone (50) mit dem ersten Beleuchtungsmittelwert E1 und dem zweiten Beleuchtungsmittelwert E2 beizutragen.

10. Vorrichtung nach einem der Ansprüche 6 bis 9, wobei die oder jede Zusatzlichtquelle (12B, 12F) eine Leuchtdiode umfasst.

11. Verfahren zur Ausgestaltung einer Beleuchtung, die eine Zahnbeleuchtungsvorrichtung nach einem der Ansprüche 1 bis 10 einsetzt, wobei das Verfahren einen Schritt des Ausgestaltens des Beleuchtungssystems (1, 100) umfasst, während dem das Beleuchtungssystem ausgestaltet wird, damit dieses gemäß mindestens einem ersten Beleuchtungsmodus betrieben wird, in dem das Beleuchtungssystem eine vorbestimmte Zahnzone (50) auf einem ersten Spektralbereich, der zwischen 370 nm und einer Bezugswellenlänge beträgt, wobei die Bezugswellenlänge zwischen 405 und 475 nm beträgt, mit einem ersten Beleuchtungsmittelwert E1 und auf einem zweiten Spektralbereich, der zwischen dieser Bezugswellenlänge und 780 nm beträgt, mit einem zweiten Beleuchtungsmittelwert E2 beleuchtet, und wobei das Verhältnis des ersten Beleuchtungsmittelwerts E1 zu dem zweiten Beleuchtungsmittelwert E2 größer als 1 und kleiner als 12 ist, wobei das Beleuchtungssystem ferner dazu ausgestaltet ist, gemäß mindestens einem zweiten Beleuchtungsmodus betrieben zu werden, der sich von dem ersten unterscheidet, wobei die Vorrichtung einen Schalter umfasst, der dazu ausgestaltet ist, das Schalten des Betriebs des Beleuchtungssystems zwischen dem ersten Beleuchtungsmodus und dem zweiten Beleuchtungsmodus zu bewirken, um die Wahrnehmung eines Benutzers der Vorrichtung nach Bedarf anzupassen und die Besonderheiten der beleuchteten Zähne zu berücksichtigen, wobei das Schalten ausgeführt wird, indem der eine der zwei Werte von dem ersten Beleuchtungsmittelwert E1 und dem zweiten Beleuchtungsmittelwert E2 verändert wird, während der andere von diesen zwei Werten unverändert bleibt, und das Beleuchtungssystem so ausgestaltet ist, dass das Verhältnis des ersten Beleuchtungsmittelwerts E1 zu dem zweiten Beleuchtungsmittelwert E2 in dem ersten Beleuchtungsmodus beziehungsweise in dem zweiten Beleuchtungsmodus unterschiedliche Werte aufweist.

## Claims

1. A dental lighting device comprising at least one lighting system (1, 100) comprising:
a supplementary instrument (1),
at least a first supplementary light source (12F) connected to this supplementary instrument (1) and configured to light a predetermined dental zone (50), over a first spectral range comprised between 370 nm and a reference wavelength, said reference wavelength being comprised between 405 nm and 475 nm, with a first average irradiance value E1, and
at least a second supplementary light source (12B) connected to this supplementary instrument (1) and configured to light a predetermined dental zone (50), over a second spectral range comprised between this reference wavelength and 780 nm, with a second average irradiance value E2,
the lighting system (1, 100) being configured to function according to at least one first lighting mode, in which the ratio of the first average irradiance value E1 to the second average irradiance value E2 is greater than 1 and less than 12, and
the lighting system being also configured to function according to at least one second lighting mode distinct from the first, the device comprising a control system of the first and the second supplementary light sources and a switch coupled to the control system and configured to operate the control system to switch operation of the lighting system between the first lighting mode and the second lighting mode in order to adjust the perception of a user of the device as needed and take into account the specificities of the teeth illuminated, the switching being realised by varying one of the two values of the first average irradiance value E1 and the second average irradiance value E2, whereas the other of these two values remains unchanged, and the lighting system being configured so that the ratio of the first average irradiance value E1 to the second average irradiance value E2 has different values in the first lighting mode and in the second lighting mode respectively.

2. The device according to Claim 1, in which the lighting system (1, 100) is configured, in the first lighting mode, to light the predetermined dental zone (50) with the first average irradiance value E1 which is greater than 1 W/m².

3. The device according to Claim 1 or 2, in which, in the first lighting mode, said ratio is less than 10.

4. The device according to Claim 1 to 3, in which the reference wavelength is between 440 nm and 460 nm.

5. The device according to any one of Claims 1 to 4, comprising an operating light (100), and in which the lighting system comprises at least one ambient light source connected to this operating light.

6. The device according to Claim 5, in which the supplementary instrument (1) comprises a body having a fixing part (11) capable of cooperating with a tool, for example a scaler, to fix said tool and the body relative to each other, and at least one housing arranged in the region of the fixing part (11) and in which said at least one supplementary light source (12B, 12F) is mounted.

7. The device according to Claim 5 or 6, in which the supplementary light sources (12B, 12F) are discrete.

8. The device according to Claim 7, in which the supplementary light sources (12B, 12F) have separate emission spectra.

9. The device according to Claim 7 or 8, in which the lighting system comprises a luminous flux mixer (13), for example a light guide, at the input of which luminous fluxes respectively emitted by the supplementary light sources (12B, 12F) are injected, and at the output of which a light beam (F) adapted to contribute in all or part to lighting the predetermined dental zone (50) emerges, with the first average irradiance value E1 and the second average irradiance value E2.

10. The device according to any one of Claims 6 to 9, in which the or each supplementary light source (12B, 12F) comprises a light-emitting diode.

11. A process for lighting configuration using a dental lighting device according to any one of Claims 1 to 10, the process comprising a configuration step of the lighting system (1, 100), during which the lighting system is configured so that the latter operates according to at least one first lighting mode, in which said lighting system lights a predetermined dental zone (50), over a first spectral range which comprised between 370 nm and a reference wavelength, said reference wavelength being comprised between 405 nm and 475 nm, with a first average irradiance value E1 and, over a second spectral range comprised between this reference wavelength and 780 nm, with a second average irradiance value E2, and in which the ratio of the first average irradiance value E1 to the second average irradiance value E2 is greater than 1 and less than 12, the lighting system being also configured to function according to at least one second lighting mode distinct from the first, the device comprising a switch configured to switch operation of the lighting system between the first lighting mode and the second lighting mode in order to adjust the perception of a user of the device as needed and take into account the specificities of the teeth illuminated, the switching being realised by varying one of the two values of the first average irradiance value E1 and the second average irradiance value E2, whereas the other of these two values remains unchanged, and the lighting system being configured so that the ratio of the first average irradiance value E1 to the second average irradiance value E2 has different values in the first lighting mode and in the second lighting mode respectively.
